# EUROPÄISCHE PATENTSCHRIFT

(11) **EP 2 752 167 B1**
(45) Veröffentlichungstag und Bekanntmachung des Hinweises auf die Patenterteilung: **01.06.2016**
(21) Anmeldenummer: 13150346.8
(22) Anmeldetag: 07.01.2013
(51) Int. Cl.: A61C 1/05, A61B 17/00

(54) **Turbinenlaufrad für ein medizinisches, insbesondere dentales, Handstück**
Turbine impeller for a medical, in particular dental handpiece
Roue directrice de turbine pour une pièce à main médicale, en particulier dentaire

(43) Veröffentlichungstag der Anmeldung: 09.07.2014
(73) Patentinhaber: W & H Dentalwerk Bürmoos GmbH, 5111 Bürmoos (AT)
(72) Erfinder: Schatz, Norbert, 5111 Bürmoos (AT); Schmiedlechner, Karl, 5121 Ostermiething (AT); Ploy, Gernot, 5111 Bürmoos (AT)

(56) Entgegenhaltungen:
- CH-A- 354 894
- GB-A- 1 169 873
- US-A- 2 871 562
- US-A1- 2010 203 472

## Beschreibung

Die vorliegende Erfindung bezieht sich auf ein Turbinenlaufrad für ein medizinisches, insbesondere dentales, Handstück nach dem Oberbegriff des Anspruchs 1.

Derartige Laufräder dienen bevorzugt zum Antrieb medizinischer, insbesondere dentaler, Behandlungswerkzeuge, welche insbesondere zur Bearbeitung von hartem oder weichem Gewebe ausgebildet sind. Hierzu sind die Laufräder vorzugsweise mit einer drehbar gelagerten Werkzeugaufnahme für das Behandlungswerkzeug verbunden und in den medizinischen Hand- oder Winkelstücken aufgenommen. Die Laufräder sind hierbei bevorzugt in den Köpfen der Hand- oder Winkelstücke angeordnet, welche insbesondere gewinkelt an den Handstückteilen angebracht sind, so dass dentale Winkelstücke vorliegen. Um das zumindest eine Laufrad in den Hand- oder Winkelstücken mit einem Antriebsfluid, insbesondere Druckluft, zu beaufschlagen, weisen die Hand- oder Winkelstücke jeweils zumindest eine Versorgungsleitung auf, welche über eine Anschlussvorrichtung bevorzugt mittels eines Versorgungsschlauches mit einer medizinischen, insbesondere dentalen, Einheit mit zumindest einer Fluidquelle verbindbar ist. Die zumindest eine Versorgungsleitung ist derart in den Hand- oder Winkelstücken angeordnet, dass deren freies Ende auf die Frontseiten der mehreren Turbinenschaufeln des Turbinenrads gerichtet ist. Durch die Anströmung der Turbinenschaufeln wird dem strömenden Fluid ein Teil seiner Energie entzogen, der auf die Schaufeln der Turbinen übergeht und die Turbinen in Drehung versetzt.

Im Stand der Technik sind eine Reihe von Turbinenrädern bekannt, welche zur Verbesserung der Antriebsleistung, verschiedenste Schaufelformen aufweisen, um eine verbesserte Abgabe der Energie des strömenden Fluides an das Turbinenrad oder eine verbesserte Umlenkung des Fluides an den Frontseiten der Turbinenschaufeln zu erzielen.

Ein derartiges Turbinenrad ist insbesondere in der DE 198 33 249 A1 beschrieben.

Das bekannte Turbinenrad ist im Wesentlichen durch einen zylindrischen Körper gebildet, der an seinem Umfang eine Mehrzahl von Schaufeln mit sich in axialer und in radialer Richtung zur Längsachse des Turbinenrads erstreckende Schaufelflächen aufweist. Die Frontseite der mehreren Schaufeln ist zur axialen Richtung der Schaufeln, insbesondere bezüglich der Einströmrichtung, konkav gekrümmt. Zur radialen Richtung der Turbinenschaufeln weist die Frontseite keine Krümmung auf. Die Rückseite der mehreren Schaufeln ist parallel zur axialen auch als zur radialen Richtung der Turbinenschaufeln ausgebildet. Diese weist somit keine Krümmungsfläche auf. Durch die Beaufschlagung der konkav gekrümmten Frontseite der mehreren Turbinenschaufeln mit einem Antriebsfluid, insbesondere mit Druckluft, wird die Energie des strömenden Fluides auf die Schaufeln der Turbinen übertragen und gleichzeitig die Abgabe der Energie durch die Umlenkung des Fluides an der Frontseite der mehreren Schaufeln erhöht.

Ein weiteres Turbinenrad ist aus der DE 198 07 566 A1 bekannt.

Dieses bekannte Turbinenrad weist eine Nabe mit mehreren Turbinenschaufeln auf, welche sich in axialer Richtung entlang der Nabe und in radialer Richtung von der Nabe erstrecken. Die Frontseite der mehreren Turbinenschaufeln ist zu der axialen Richtung der Schaufeln gekrümmt ausgebildet. Zur radialen Richtung der Turbinenschaufeln weist die Frontseite keine Krümmung auf. Die konvex gekrümmte Rückseite der mehreren Schaufeln ist sowohl in der axialen auch als in der radialen Richtung parallel zu der konkav gekrümmten Frontseite ausgebildet. Auch durch diese Ausgestaltung des Turbinenrads wird die Energie des strömenden Fluides auf die Schaufeln der Turbinen übertragen und gleichzeitig die Abgabe der Energie durch die Umlenkung des Fluides an der Frontseite der mehreren Schaufeln erhöht.

Weitere Turbinenräder mit Turbinenschaufeln, welche eine konvex gekrümmte Rückseite aufweisen, sind auch aus der GB 1 169 873 A, der US 2 871 562 A und der CH 354 894 A bekannt.

Die US 2010/203472 A1 offenbart ein weiteres Turbinenrad mit mehreren Schaufeln, bei denen lediglich die axialen Enden der Rückseiten eine bevorzugt konvex ausgebildete Neigungsfläche aufweisen.

Bei den aus dem Stand der Technik bekannten Turbinenrädern ist zwar eine verbesserte Abgabe der Energie des strömenden Fluides an die Laufräder sowie eine verbesserte Umlenkung des Fluides an den Schaufeln, insbesondere durch die zur axialen Richtung der Schaufeln konkav ausgebildeten Frontseiten, beschrieben, eine verbesserte Rückführung des Antriebsfluid von den Turbinenschaufeln, insbesondere vorbei an den Rückseiten der benachbarten Turbinenschaufeln wird jedoch nicht offenbart.

Der vorliegenden Erfindung liegt nun die Aufgabe zugrunde die Leistung des Turbinenantriebs, insbesondere bei niedrigen Drehzahlen, zu erhöhen, um die Baugröße des Turbinenrads und das, das Laufrad aufnehmende, Gehäuseteil des medizinischen, insbesondere dentalen, Hand- oder Winkelstücks zu verkleinern.

Zur Lösung dieser Aufgabe weist das Turbinenrad für ein fluidbetriebenes medizinisches, insbesondere dentales, Handstück gemäß einem ersten Ausführungsbeispiel eine Nabe sowie mehrere Turbinenschaufeln auf, welche sich in axialer Richtung entlang der Nabe und in radialer Richtung von der Nabe erstrecken, wobei die Rückseite der Turbinenschaufeln jeweils zumindest eine konkave Fläche aufweist, welche zur Frontseite der Turbinenschaufeln gekrümmt ist. Bevorzugt ist die zumindest eine konkave Fläche der Rückseite zu der axialen und/ oder radialen Richtung der Turbinenschaufeln gekrümmt.

Gemäß einem zweiten Ausführungsbeispiel schneidet die konkave Fläche der Rückseite zumindest ein axiales Ende der Turbinenschaufeln, um eine Ausnehmung zur Rückführung der Treibluft zu schaffen.

Gemäß einem weiteren Ausführungsbeispiel weist die Frontseite der Turbinenschaufeln jeweils zumindest eine konkave Fläche auf, welche zur Rückseite der Turbinenschaufeln gekrümmt ist. Diese Fläche ist vorzugsweise ebenfalls zu der axialen und/ oder radialen Richtung der Turbinenschaufeln gekrümmt, wobei die Krümmung der Frontseiten der Turbinenschaufeln bevorzugt in axialer Richtung und/ oder radialer Richtung zur Nabe hin zunehmend ausgebildet ist.

Gemäß einem weiteren Ausführungsbeispiel weist die Rückseite der Turbinenschaufeln zumindest an einem axialen und/ oder radialen Ende eine konvexe Fläche auf. Die zumindest eine konvexe Fläche ist hierbei zu der axialen und/ oder radialen Richtung der Turbinenschaufeln gekrümmt. Die Krümmung zur axialen und/ oder radialen Richtung der Turbinenschaufeln ist hierbei bevorzugt derart ausgebildet, dass die konvex gekrümmte Fläche von der Frontseite weggeneigt ist.

Gemäß einem weiteren Ausführungsbeispiel sind die mehreren Turbinenschaufeln zumindest an einem axialen Ende über einen Steg miteinander verbunden, wobei die den Turbinenschaufeln zugewandte Seite des Stegs bevorzugt zumindest eine konkave Fläche aufweist. Die Front- und/ oder Rückseite der mehreren Turbinenschaufeln verlaufen hierbei bevorzugt tangential in den Steg. Des Weiteren verläuft die Frontseite der Turbinenschaufeln bevorzugt tangential in die Rückseite der benachbarten Turbinenschaufeln.

Gemäß einem weiteren Ausführungsbeispiel weist die Nabe des Turbinenlaufrads zumindest an einem axialen Ende der Turbinenschaufeln einen Vorsprung auf, um die mehreren Schaufeln beabstandet von angrenzenden Bauteilen in dem medizinischen, insbesondere dentalen, Handstück zu lagern.

Gemäß eines Ausführungsbeispiel des medizinischen, insbesondere dentalen, Hand- oder Winkelstücks, weist dieses ein Turbinenlaufrad gemäß einem der oben angeführten Ausführungsbeispiele auf.

Im Rahmen der Erfindung versteht es sich selbstverständlich, dass das erfindungsgemäße Turbinenrad nicht auf die oben beschriebenen Ausführungsbeispiele und auf die Verwendung bei einem medizinischen, insbesondere dentalen, Hand- oder Winkelstück gemäß den oben angeführten Ausführungsbeispiel beschränkt ist. Das Turbinenrad kann neben der Verwendung bei einem medizinischen, insbesondere dentalen Hand- oder Winkelstück mit einer bevorzugt drehbar gelagerten Werkzeugaufnahme für ein Behandlungswerkzeug, wobei das Turbinenlaufrad mit der Werkzeugaufnahme verbunden ist, bei einem elektromechanischen Wandler, insbesondere bei einem elektrischen Generator, zum Einsatz kommen, wobei das Laufrad zur Wandlung der Energie eines strömenden Fluides in elektrische Energie dient. Des Weiteren kann das Turbinenrad in einer medizinischen, insbesondere dentalen, Antriebseinheit zum Antrieb eines mit der Antriebseinheit verbundenen medizinischen, insbesondere dentalen, Instruments oder einer medizinischen, insbesondere dentalen Kupplungsvorrichtung zur Verbindung eines medizinischen Hand- oder Winkelstücks mit einem Versorgungsschlauch zum Einsatz kommen.

Das vorliegende Turbinenrad zeichnet sich durch eine Reihe erheblicher Vorteile aus.

Durch die Ausbildung der Rückseite mit zumindest einer konkav gekrümmten Fläche, welche zur Frontseite der Turbinenschaufeln gekrümmt ist, weisen die Schaufeln eine strömungsgünstigere Form auf, als die im Stand der Technik bekannten Turbinenräder, da dem Antriebsfluid nach dessen Umlenkung an den Frontseiten der Turbinenschaufeln an den Rückseiten der angrenzenden Schaufeln weniger Widerstand entgegensteht. Durch die Ausnehmung, insbesondere durch die konkave Fläche an den Rückseiten, wird für den umgelenkten Fluidstrom mehr Platz zwischen den mehreren Turbinenschaufeln geschaffen. Die Energie des strömenden Fluides kann somit besser ausgenutzt werden, wodurch eine größere nutzbare Leistung erzielt werden kann.

Einen weiteren Vorteil der Erfindung bildet die Ausgestaltung der Front- und Rückseiten der mehreren Turbinenschaufeln in Kombination mit dem Steg, welcher die mehreren Schaufeln miteinander verbindet. Durch die Ausbildung der Front- und Rückseiten der Schaufeln sowie die den mehreren Schaufeln zugewandte Innenseite des Stegs durch eine konkave Fläche kann ebenfalls die Energie des strömenden Fluides besser ausgenutzt werden. Das strömende Fluid findet an den konkaven Frontseiten der Schaufeln einen größeren Widerstand, wobei gleichzeitig eine verbesserte Umlenkung des strömenden Fluides durch die konkav gekrümmte Innenseite des Stegs und eine verbesserte Rückführung des Antriebsfluid vorbei an den Rückseiten der Turbinenschaufeln durch die konkav gekrümmte Fläche in der Rückseite der Schaufeln erfolgt.

Nachfolgend wird die Erfindung anhand mehrerer Ausführungsbeispiele und in Verbindung mit den beigefügten Zeichnungen erläutert.

### Dabei zeigt:

Figur 1 das Turbinenrad in einer perspektivischen Darstellung,
Figur 2 eine perspektivische Darstellung des Turbinenrads mit einem Schnitt durch eine radiale Querschnittsebene AA,
Figur 3 das Turbinenrad in der Seitendarstellung, sowie
Figur 4 das Turbinenrad in der Draufsicht mit einem Schnitt durch die radiale Querschnittsebene AA aus Figur 2;

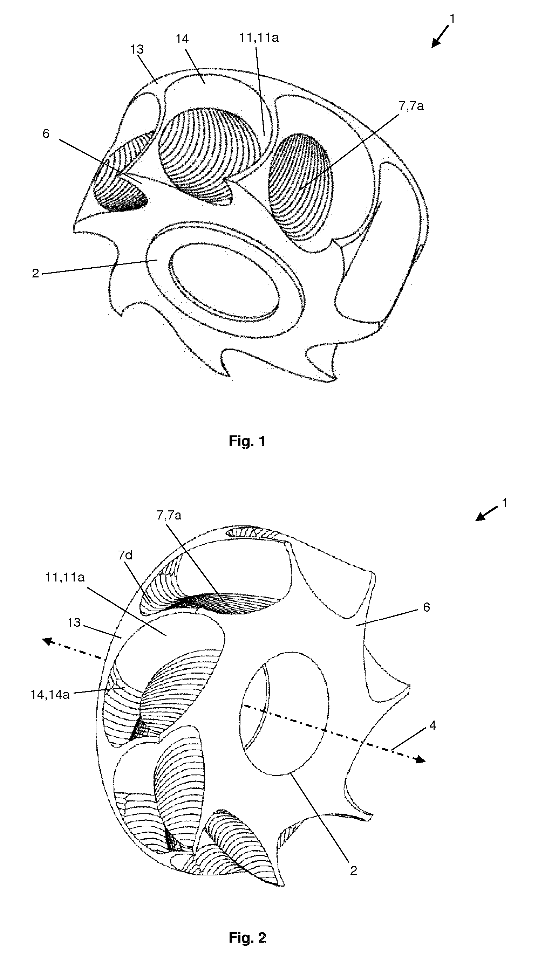

Das in Figur 1 dargestellt Turbinenlaufrad 1 ist im Wesentlichen durch einen bevorzugt zylindrisch ausgebildeten Körper gebildet, durch welchen sich eine Nabe 2 erstreckt. Diese ist bevorzugt hohl ausgebildet, um eine drehbar gelagerte Werkzeugaufnahme für ein Behandlungswerkzeug aufzunehmen. In radialer Richtung zur Nabe 2 erstrecken sich mehrere Turbinenschaufeln 6. Die Rückseite 7 der mehreren Turbinenschaufeln 6 weist jeweils zumindest eine konkave Fläche 7a auf, welche zur Frontseite 11 der Turbinenschaufeln 6 gekrümmt ist. Die konkave Fläche 7a ist hierbei bevorzugt zu der axialen und radialen Richtung der Turbinenschaufeln 6 gekrümmt ausgebildet, wodurch eine sphärische Fläche 7a, insbesondere eine kalottenförmige Ausnehmung, zur Rückführung der Treibluft gebildet wird. Die Frontseite 11 der mehreren Turbinenschaufeln 6 weist ebenfalls jeweils zumindest eine konkave Fläche 11a auf, welche zur Rückseite 7 der Turbinenschaufeln 6 gekrümmt ausgebildet ist. Die gekrümmte Fläche 11a ist hierzu zu der axialen und radialen Richtung der Turbinenschaufeln 6 gekrümmt, wodurch die Frontseite 11 eine sphärisch ausgebildete Fläche 11a, insbesondere eine kugelsegmentförmige Oberfläche, aufweist. Beide gekrümmten Flächen 7a, 11a der Front- und Rückseite 7, 11 der mehreren Schaufeln 6 sind somit bevorzugt in axialer und radialer Richtung zumindest teilweise bikonkav zueinander ausgebildet.

Zumindest an einem axialen Ende sind in diesem Ausführungsbeispiel die mehreren Turbinenschaufeln 6 über einen Steg 13 miteinander verbunden, wobei die den Turbinenschaufeln 6 zugewandte Seite 14 des Stegs 13 die Frontseite 11 der mehreren Schaufeln 6 mit der Rückseite 7 der benachbarten Schaufeln 6 verbindet.

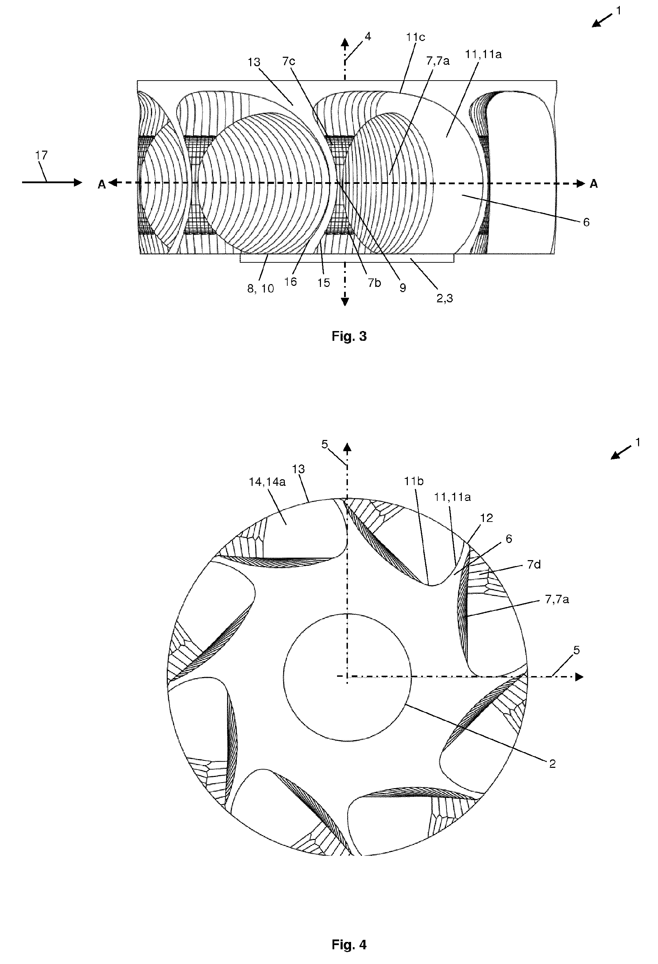

Figur 2 zeigt eine perspektivische Darstellung des Turbinenrad 1 mit einem Schnitt durch eine radiale Querschnittsebene AA, wie in Figur 3 gezeigt. Wie bereits in Figur 1 abgebildet, weist das Turbinenrad 1 eine Nabe 2 mit mehreren Turbinenschaufeln 6 auf, welche sich in axialer Richtung 4 entlang der Nabe 2 und in radialer Richtung von der Nabe 2 erstrecken. Die Front- und Rückseite 7, 11 der mehreren Schaufeln 6 weisen jeweils zumindest eine konkave Fläche 7a, 11a auf, welche bevorzugt zur axialen und radialen Richtung der Turbinenschaufeln 6 gekrümmt ausgebildet ist. Wie in der Querschnittsebene AA gezeigt, ist die konkave Fläche 7a der Rückseite 7 zu der Frontseite 11 gekrümmt ausgebildet und weist in der Ebene AA die stärkste Krümmung zur radialen Richtung der Turbinenschaufeln 6 auf. Die kalottenförmige Ausnehmung weist somit in der Ebene AA den tiefsten Punkt auf.

Neben der konkaven Fläche 7a weisen die mehreren Schaufeln 6 an der Rückseite 7 jeweils zumindest eine erste Übergangsfläche 7d auf, welche tangential in die Innenseite 14 des Stegs 13 verläuft. Die den Turbinenschaufeln 6 zugewandte Seite 14 des Stegs 13 ist hierzu bevorzugt durch zumindest eine konkave Fläche 14a gebildet.

In Figur 3 ist das Turbinenrad 1 aus den Figuren 1 und 2 in einer Seitendarstellung gezeigt. Das Turbinenrad 1 weist an der Rückseite 7 der Turbinenschaufeln 6, neben der zumindest einen konkaven Fläche 7a, welche bevorzugt zur axialen Richtung 4 und zur radialen Richtung der Turbinenschaufeln 6 gekrümmt ist, zumindest an einem axialen und radialen Ende 8, 9 eine konvexe Fläche 7b bzw. 7c auf. Die konvexe Fläche 7b bzw. 7c der Rückseite 7 ist hierbei bevorzugt zu der axialen und radialen Richtung der Turbinenschaufeln 6 gekrümmt. Insbesondere ist die Krümmung der konvexen Flächen 7b, 7c derart ausgebildet, dass diese von der Frontseite 11, insbesondere von der gekrümmten Fläche 11a, weggeneigt ist. Wie die Stirnfläche 12 der Turbinenschaufeln 6 zeigt, laufen die Kanten 15 und 16, welche durch die Front- und Rückseite 7, 11 gebildet sind, mit zunehmenden Abstand von der radialen Querschnittsebene AA auseinander. Der Abstand zwischen beide Kanten 15 und 16 nimmt somit in axialer Richtung 4 zu.

In einem oberen axialen Ende sind die mehreren Schaufeln 6 über den Steg 13 miteinander verbunden. An dem zweiten axialen Ende 8 weist das Turbinenrad 1, bzw. die Nabe 2, einen Vorsprung 3 auf, um die mehreren Schaufeln 6 beabstandet von angrenzenden Bauteilen in dem medizinischen, insbesondere dentalen, Handstück zu lagern.

Der Pfeil 17 stellt die bevorzugte Einströmrichtung 17 für das Antriebsfluid dar. Hierbei ist die Versorgungsleitung für das Antriebsfluid bevorzugt senkrecht zur axialen Richtung 4 des Turbinenrads 1 ausgerichtet. Des Weiteren erfolgt die Zuführung des Antriebsfluides bevorzugt in einem mittleren axialen Bereich AA des Turbinenrads 1. Das zugeführte Antriebsfluid trifft hierbei auf die Frontseite 11 der mehreren Schaufeln 6 des Turbinenrads 1. Die Frontseite 11 der mehreren Schaufeln 6 ist hierzu jeweils dazu ausgebildet das zugeführte Antriebsfluid zu empfangen und an die Rückseite 7 der bevorzugt benachbarten Schaufeln 6 weiterzuleiten. Die Rückseiten 7 wiederum sind derart ausgebildet, das Antriebsfluid von der Frontseite 11 der mehreren Schaufeln 6 zu empfangen und von den Schaufeln 6, insbesondere in axialer und/ oder radialer Richtung, von dem Turbinenrad 1 wegzuleiten. Hierzu ist die konkave Fläche 7a bevorzugt derart an der Rückseite 7 der Turbinenschaufeln 6 angebracht, dass die Fläche 7a zur Einströmrichtung 17 konkav gekrümmt ist und im mittleren axialen Bereich AA des Turbinenrads angeordnet ist. Durch diese Ausbildung der Rückseite 7 der Turbinenschaufeln 6 mit der zumindest einen konkaven Fläche 7a im mittleren axialen Bereich AA der Schaufeln 6, welche zur Frontseite 11 der Turbinenschaufeln 6 gekrümmt ist, wird für den Fluidstrom mehr Platz zwischen den mehreren Turbinenschaufeln 6 geschaffen, so dass diesem, auf Grund der kalottenförmige Ausnehmung, gebildet durch die konkave Fläche 7a, weniger Widerstand entgegensteht. Insbesondere wird der Abstand zwischen der Frontseite 11 und der Rückseite 7 einer benachbarten Schaufel 6, insbesondere der dadurch gebildete Schaufelraum für das Antriebsfluid, bevorzugt im mittleren axialen Bereich AA vergrößert.

Figur 4 zeigt das Turbinenrad 1 mit einem Schnitt durch die radiale Querschnittsebene AA in einer Draufsicht. Die Frontseite 11 der Turbinenschaufeln 6 mit der konkaven Fläche 11a, welche zur radialen Richtung 5 der Turbinenschaufeln 6 gekrümmt ausgebildet ist, weist eine Übergangsfläche 11b auf, die tangential in die Rückseite 7 der benachbarten Turbinenschaufeln verläuft. Hierbei nimmt die Krümmung der Fläche 11 a bzw. 11 b in radialer Richtung 5 zur Nabe 2 und mit zunehmenden Abstand von der Stirnfläche 12 zu. Die Rückseite 7 mit der zumindest einen konkav ausgebildeten Fläche 7a, welche ebenfalls zur radialen Richtung 5 der Turbinenschaufeln 6 gekrümmt ausgebildet ist, weist in diesem Ausführungsbeispiel eine zweite konkav gekrümmte Übergangsfläche 7d auf, welche in die bevorzugt konkav ausgebildete Fläche 14a der den Schaufeln zugewandten Innenseite 14 des Stegs 13 verläuft. Die konkav gekrümmte Übergangsfläche 7d ist hierbei in axialer und radialer Richtung zu den Turbinenschaufeln 6 gekrümmt ausgebildet.

Gemäß dem bevorzugten Ausführungsbeispiel, wie in den Figuren 1 bis 4 beschrieben, weist somit das Turbinenrad 1 eine Frontseite 11 auf, welche eine konkave Fläche 11 a, die zur Rückseite 7 der Turbinenschaufeln 6 gekrümmt ausgebildet ist, und eine Übergangsfläche 11b umfasst, die tangential in die Rückseite 7 der benachbarten Turbinenschaufeln 6 verläuft. Die Rückseite 7 der mehreren Schaufeln 6 weist eine konkave Fläche 7a, welche zur Frontseite 11 der Turbinenschaufeln 6 gekrümmt ausgebildet ist, zumindest eine konvexe Fläche 7b bzw. 7c sowie eine zweite konkav gekrümmte Übergangsfläche 7d auf. Die mehreren Schaufeln 6 sind über einen Steg 13 miteinander verbunden, welcher eine Innenseite 14 mit einer konkaven Fläche 14a umfasst. Die konkav oder konvex gekrümmten Flächen 7a, 7b, 7c, 7d, 11a, 11b der Front- und Rückseite 7, 11 der Turbinenschaufeln 6 sind bevorzugt zu der axialen und radialen Richtung der Turbinenschaufeln 6 gekrümmt ausgebildet. Somit umfasst weder die Front- noch die Rückseite ebene, insbesondere plane, Flächen.

## Patentansprüche

1. Turbinenlaufrad (1) für ein fluidbetriebenes medizinisches, insbesondere dentales, Handstück aufweisend, eine Nabe (2) sowie mehrere Turbinenschaufeln (6), welche sich in axialer Richtung (4) entlang der Nabe (2) und in radialer Richtung (5) von der Nabe (2) erstrecken, **dadurch kennzeichnet, dass** die Rückseite (7) der Turbinenschaufeln (6) jeweils zumindest eine konkave Fläche (7a), welche zur Frontseite (11) der Turbinenschaufeln (6) gekrümmt ist, aufweist.

2. Turbinenlaufrad (1) nach Anspruch 1, **dadurch gekennzeichnet, dass** die zumindest eine konkave Fläche (7a) der Rückseite (7) zu der axialen und/ oder radialen Richtung (4, 5) der Turbinenschaufeln (6) gekrümmt ist.

3. Turbinenlaufrad (1) nach Anspruch 1 oder 2, **dadurch gekennzeichnet, dass** die konkave Fläche (7a) der Rückseite (7) zumindest ein axiales Ende (8) der Turbinenschaufeln (6) schneidet, so dass die Rückseite (7) eine Ausnehmung (10) zur Rückführung der Treibluft aufweist.

4. Turbinenlaufrad (1) nach einem der vorherigen Ansprüche, **dadurch gekennzeichnet, dass** die Frontseite (11) der Turbinenschaufeln (6) jeweils zumindest eine konkave Fläche (11a), welche zur Rückseite (7) der Turbinenschaufeln (6) gekrümmt ist, aufweist.

5. Turbinenlaufrad (1) nach Anspruch 4, **dadurch gekennzeichnet, dass** die zumindest eine konkave Fläche (11a) der Frontseite (11) zu der axialen und/ oder radialen Richtung (4, 5) der Turbinenschaufeln (6) gekrümmt ist.

6. Turbinenlaufrad (1) nach Anspruch 5, **dadurch gekennzeichnet, dass** die Krümmung der Frontseiten (11) der Turbinenschaufeln (6) in axialer Richtung (4) und/ oder radialer Richtung (5) zur Nabe (2) zunehmend ausgebildet ist.

7. Turbinenlaufrad (1) nach einem der vorherigen Ansprüche, **dadurch gekennzeichnet, dass** die Rückseite (7) der Turbinenschaufeln (6) zumindest an einem axialen und/ oder radialen Ende (8, 9) eine konvexe Fläche (7b, 7c) aufweist.

8. Turbinenlaufrad (1) nach Anspruch 7, **dadurch gekennzeichnet, dass** die zumindest eine konvexe Fläche (7b, 7c) der Rückseite (7) zu der axialen und/ oder radialen Richtung (4, 5) der Turbinenschaufeln (6) gekrümmt ist.

9. Turbinenlaufrad (1) nach Anspruch 7 oder 8, **dadurch gekennzeichnet, dass** die Krümmung der konvexen Fläche (7b, 7c) derart ausgebildet ist, dass diese von der Frontseite (11) weggeneigt ist.

10. Turbinenlaufrad (1) nach einem der vorherigen Ansprüche, **dadurch gekennzeichnet, dass** die mehreren Turbinenschaufeln (6) zumindest an einem axialen Ende über einen Steg (13) miteinander verbunden sind.

11. Turbinenlaufrad (1) nach Anspruch 10, **dadurch gekennzeichnet, dass** die den Turbinenschaufeln (6) zugewandte Seite (14) des Stegs (13) zumindest eine konkave Fläche (14a) aufweist.

12. Turbinenlaufrad (1) nach Anspruch 10 oder 11, **dadurch gekennzeichnet, dass** die Front- und/ oder Rückseite (7, 11) der mehreren Turbinenschaufeln (6) tangential in den Steg (13) verlaufen.

13. Turbinenlaufrad (1) nach einem der vorherigen Ansprüche, **dadurch gekennzeichnet, dass** die Frontseite (11) der Turbinenschaufeln (6) tangential in die Rückseite (7) der benachbarten Turbinenschaufeln (6) verläuft.

14. Turbinenlaufrad (1) nach einem der vorherigen Ansprüche, **dadurch gekennzeichnet, dass** die Nabe (2) zumindest an einem axialen Ende (8) der Turbinenschaufeln (6) einen Vorsprung (3) aufweist, um die mehreren Schaufeln (6) beabstandet von angrenzenden Bauteilen in dem medizinischen, insbesondere dentalen, Handstück zu lagern.

15. Medizinisches, insbesondere dentales, Hand- oder Winkelstück, **gekennzeichnet durch** ein Turbinenlaufrad (1) nach einem der Ansprüche 1 bis 14.

## Claims

1. A turbine impeller (1) for a fluid-operated medical, in particular dental, handpiece having a hub (2) and a plurality of turbine blades (6), which extend in the axial direction (4) along the hub (2) and in the radial direction (5) away from the hub (2), **characterized in that** the rear side (7) of the turbine blades (6) has at least one concave surface (7a), which is curved toward the front side (11) of the turbine blades (6).

2. The turbine impeller (1) according to claim 1, **characterized in that** the at least one concave surface (7a) of the rear side (7) is curved toward the axial and/or radial direction (4, 5) of the turbine blades (6).

3. The turbine impeller (1) according to claim 1 or 2, **characterized in that** the concave surface (7a) of the rear side (7) intersects at least one axial end (8) of the turbine blades (6), so that the rear side (7) comprises a recess (10) for recirculation of the impelling air.

4. The turbine impeller (1) according to any one of the preceding claims, **characterized in that** the front side (11) of the turbine blades (6) comprises at least one concave surface (11a), which is curved toward the rear side (7) of the turbine blades (6).

5. The turbine impeller (1) according to claim 4, **characterized in that** the at least one concave surface (11a) of the front side (11) is curved toward the axial and/or radial directions (4, 5) of the turbine blades (6).

6. The turbine impeller (1) according to claim 5, **characterized in that** the curvature of the front sides (11) of the turbine blades (6) is designed to increase in the axial direction (4) and/or in the radial direction (5) toward the hub (2).

7. The turbine impeller (1) according to any one of the preceding claims, **characterized in that** the rear side (7) of the turbine blades (6) has a convex surface (7b, 7c) at least on an axial and/or radial end (8, 9).

8. The turbine impeller (1) according to claim 7, **characterized in that** the at least one convex surface (7b, 7c) of the rear side (7) is curved toward the axial and/or radial directions (4, 5) of the turbine blades (6).

9. The turbine impeller (1) according to claim 7 or 8, **characterized in that** the curvature of the convex surface (7b, 7c) is designed so that it is inclined away from the front side (11).

10. The turbine impeller (1) according to any one of the preceding claims, **characterized in that** at least on an axial end the plurality of turbine blades (6) are connected to one another by a web (13).

11. The turbine impeller (1) according to claim 10, **characterized in that** the side (14) of the web (13) facing the turbine blades (6) comprises at least one concave surface (14a).

12. The turbine impeller (1) according to claim 10 or 11, **characterized in that** the front side and/or rear side (7, 11) of the plurality of turbine blades (6) run(s) tangentially into the web (13).

13. The turbine impeller (1) according to any one of the preceding claims, **characterized in that** the front side (11) of the turbine blades (6) runs tangentially into the rear side (7) of the adjacent turbine blades (6).

14. The turbine impeller (1) according to any one of the preceding claims, **characterized in that** the hub (2) comprises a protrusion (3) at least on an axial end (8) of the turbine blades (6) to support the plurality of blades (6) at a distance from the adjacent components in the medical, in particular dental, handpiece.

15. A medical, in particular dental, handpiece or contra-angle handpiece, **characterized by** a turbine impeller (1) according to any one of claims 1 to 14.

## Revendications

1. Roue de turbine (1) pour une pièce à main médicale, en particulier dentaire, entraînée par fluide, présentant un moyeu (2) ainsi que plusieurs ailettes de turbine (6) qui s'étendent en direction axiale (4) le long du moyeu (2) et en direction radiale (5) à partir du moyeu (2), **caractérisée en ce que** la face arrière (7) des ailettes de turbine (6) présente respectivement au moins une surface concave (7a), laquelle est incurvée vers la face frontale (11) des ailettes de turbine (6).

2. Roue de turbine (1) selon la revendication 1, **caractérisée en ce que** l'au moins une surface concave (7a) de la face arrière (7) est incurvée vers la direction axiale et/ou radiale (4, 5) des ailettes de turbine (6).

3. Roue de turbine (1) selon la revendication 1 ou 2, **caractérisée en ce que** la surface concave (7a) de la face arrière (7) coupe au moins une extrémité axiale (8) des ailettes de turbine (6) de manière à ce que la face arrière (7) présente un évidement (10) pour la remise en circulation de l'air propulseur.

4. Roue de turbine (1) selon l'une des revendications précédentes, **caractérisée en ce que** la face frontale (11) des ailettes de turbine (6) présente respectivement au moins une surface concave (11a) qui est incurvée vers la face arrière (7) des ailettes de turbine (6).

5. Roue de turbine (1) selon la revendication 4, **caractérisée en ce que** l'au moins une surface concave (11a) de la face frontale (11) est incurvée vers la direction axiale et/ou radiale (4, 5) des ailettes de turbine (6).

6. Roue de turbine (1) selon la revendication 5, **caractérisée en ce que** l'incurvation des faces frontales (11) des ailettes de turbine (6) est réalisée en augmentant en direction axiale (4) et/ou radiale (5) au moyeu (2).

7. Roue de turbine (1) selon l'une des revendications précédentes, **caractérisée en ce que** la face arrière (7) des ailettes de turbine (6) présente, au moins sur une extrémité axiale et/ou radiale (8, 9), une surface convexe (7b, 7c).

8. Roue de turbine (1) selon la revendication 7, **caractérisée en ce que** l'au moins une surface convexe (7b, 7c) de la face arrière (7) est incurvée vers la direction axiale et/ou radiale (4, 5) des ailettes de turbine (6).

9. Roue de turbine (1) selon la revendication 7 ou 8, **caractérisée en ce que** l'incurvation de la surface convexe (7b, 7c) est réalisée de telle sorte que celle-ci est inclinée en éloignement de la face frontale (11).

10. Roue de turbine (1) selon l'une des revendications précédentes, **caractérisée en ce que** les plusieurs ailettes de turbine (6) sont reliées entre elles, au moins à une extrémité axiale, via une nervure (13).

11. Roue de turbine (1) selon la revendication 10, **caractérisée en ce que** le côté (14) de la nervure (13), tourné vers les ailettes de turbine (6), présente au moins une surface concave (14a).

12. Roue de turbine (1) selon la revendication 10 ou 11, **caractérisée en ce que** la face frontale et/ou arrière (7, 11) des plusieurs ailettes de turbine (6) se fondent de manière tangentielle dans la nervure (13).

13. Roue de turbine (1) selon l'une des revendications précédentes, **caractérisée en ce que** la face frontale (11) des ailettes de turbine (6) se fond de manière tangentielle dans la face arrière (7) des ailettes de turbine (6) voisines.

14. Roue de turbine (1) selon l'une des revendications précédentes, **caractérisée en ce que** le moyeu (2) présente une saillie (3), au moins à une extrémité axiale (8) des ailettes de turbine (6), afin de positionner les plusieurs ailettes (6) de manière espacée par rapport à des éléments constitutifs limitrophes dans la pièce à main médicale, en particulier dentaire.

15. Pièce à main ou pièce à main à contre-angle médicale, en particulier dentaire, **caractérisée par** une roue de turbine (1) selon l'une des revendications 1 à 14.
